(19) Europäisches Patentamt
European Patent Office
Office européen des brevets

(11) Publication number: **0 391 462 A1**

# (12) EUROPEAN PATENT APPLICATION

(21) Application number: **90200719.4**

(22) Date of filing: **27.03.90**

(51) Int. Cl.[5]: **A61K 45/06, A61K 37/64, //(A61K45/06,31:505), (A61K37/64,31:505)**

(30) Priority: **05.04.89 US 333471**

(43) Date of publication of application: **10.10.90 Bulletin 90/41**

(84) Designated Contracting States: **AT BE CH DE DK ES FR GB GR IT LI LU NL SE**

(71) Applicant: **JANSSEN PHARMACEUTICA N.V.**
**Turnhoutseweg 30**
**B-2340 Beerse(BE)**

(72) Inventor: **Assogna, Giuseppe**
**Via Cechov 107**
**Rome(IT)**
Inventor: **Lucchetti, Giovanni**
**Via Mengoni 7**
**Rome(IT)**

(54) **Synergistic compositions containing ketanserin.**

(57) Pharmaceutical compositions comprising ketanserin and an ACE inhibitor in a quantity producing a synergistic antihypertensive effect. A process of preparing such synergistic compositions.

EP 0 391 462 A1

# SYNERGISTIC COMPOSITIONS CONTAINING KETANSERIN

## Background of the invention.

Several classes of chemical compounds are known as antihypertensives. Among these classes a group of piperidinylalkyl quinazolinediones having antiserotoninergic activity is of particular interest. These compounds inhibit serotonin-induced vasoconstriction and are useful as antihypertensives. Another class of known antihypertensives are the angiotensin converting enzyme (ACE) inhibitors, which exert their influence by inhibiting ACE, the enzyme mediating the conversion of angiotensin I to angiotensin II, a vasocostrictive hormone, and the degradation of the vasodilator kinin peptide (bradykinin) to its inactive metabolites.

Further, there are known antihypertensive compositions comprising ACE inhibitors and other antihypertensive agents, said compositions showing an additive effect on the lowering of the blood pressure. For example, in U.S. Pat. No. 4,217,347 there are described compositions comprising ACE inhibitors and diuretics and in EP-0,288,732, compositions of ACE inhibitors with calcium channel blockers. The antihypertensive effect of ketanserin and captopril was reported on the International Symposium on Serotonin : From cell biology to pharmacology and therapeutics, Florence, Italy, March 29 - April 1,1989.

It has now been found that the compound ketanserin in combination with ACE inhibitors synergistically acts on the lowering of the blood pressure.

## Description of the invention.

The present invention is concerned with synergistic compositions comprising a pharmaceutically acceptable carrier and as active ingredients an antihypertensively effective amount of the compound ketanserin or a pharmaceutically acceptable acid addition salt form thereof and an ACE inhibitor or a pharmaceutically acceptable acid or base addition salt form thereof.

Ketanserin as referred to hereinabove is the generic name of the compound 3-[2-[4-(4-fluorobenzoyl)-1-piperidinyl]ethyl]-2,4(1H,3H)-quinazolinedione, which may be represented by the formula

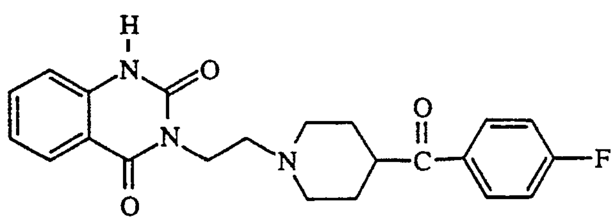

(I)

This compound, its synthesis as well as its antiserotoninergic properties are described in U.S. Pat. No. 4,335,127, incorporated herein by reference.

The term ACE inhibitor as used hereinabove defines chemical compounds which inhibit angiotensin converting enzyme (ACE), also known as kinase II. Typical examples of ACE inhibitors are the compounds mentioned in U.S. Pat. Nos 4,399,136, 4,487,929,4,374,131,4,416,831,4,473,575, Eur. Pat. No. 0,072,352; in particular benazepril; U.S. Pat. No. 4,046,889, in particular captopril; Eur. Pat. No. 0,012,401, in particular enalapril, enalaprilat and lisinopril; U.S. Pat. No. 4,248,883, in particular alacepril; U.S. Pat. Nos 4,385,051 and 4,474,692, in particular delapril, Great Britain Pat. No. 2,102,412, in particular moveltipril; U.S. Pat. No. 4,727,160, in particular ramipril and ramiprilat; U.S. Pat. No. 4,512,924, in particular cilazapril and and cilazaprilat; U.S. Pat. No. 4,344,949, in particular quinapril and quinaprilat; Eur. Pat. No. 0,063,896, in particular fosinopril; Eur. J. Clin. Pharmacol., 1987, 31, 519, in particular perindopril and perindoprilat; and also the compounds indolapril, spirapril, spiraprilat and zofenopril. These groups of active ingredients are listed with the purpose of providing representative examples and are not intended to restrict the scope of the present invention.

Particular compositions are those synergistic compositions wherein the ACE inhibitor is one of the agents pertaining to the patents or publication cited hereinabove, and more in particular the agents specifically mentioned hereinabove.

A first group of more particular compositions comprises those particular compositions wherein the ACE inhibitor is selected from captopril, lisinopril, enalapril and cilazapril.

Another group of more particular compositions comprises those particular compositions wherein the ACE inhibitor is a compound that contains the moiety

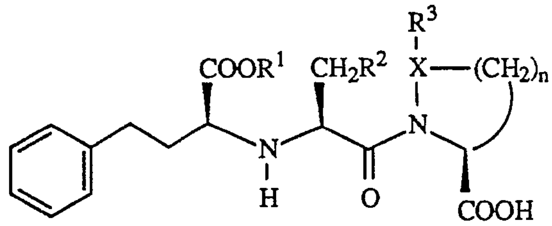

wherein $R^1$ is hydrogen or ethyl;
$R^2$ is hydrogen or aminopropyl;
$R^3$ is hydrogen; or $R^2$ and $R^3$ taken together form 1,2-ethanediyl;
X is CH or N; and
n is 2 or 3.

The active ingredient ketanserin (I) may be present in the free base form or in a pharmaceutically acceptable acid addition salt form, the latter being obtained by reaction of the base form with an appropriate acid. Appropriate acids comprise, for example, inorganic acids such as hydrohalic acids, e.g. hydrochloric or hydrobromic acid; sulfuric acid; nitric acid; phosphoric acid and the like; or organic acids such as, for example, acetic, propanoic, hydroxyacetic, 2-hydroxypropanoic, 2-oxopropanoic, ethanedioic, propanedioic, butanedioic, (Z)-butenedioic, (E)-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. A particularly interesting acid addition salt form of the compound ketanserin (I) is (+)-3-[2-[4-(4-fluorobenzoyl)-1-piperidinyl]-ethyl]-2,4(1H,3H)-quinazolinedione R-(R*,R*)]-2,3-dihydroxybutanedioate (1:1).

The ACE inhibitor may be present in its neutral or zwitterionic form or in a pharmaceutically acceptable acid or base addition salt form, the latter being obtained by reaction of the neutral or zwitterionic form with respectively an appropriate acid as defined hereinabove, or with an appropriate base. Appropriate base addition salt forms comprise, for example, the ammonium salts, the alkali and earth alkaline metal salts, e.g., the sodium, potassium, magnesium, calcium salts and the like, salts with organic bases, e.g., the benzathine, N-methyl-D-glucamine, hydrabamine salts, and salts with amino acids such as, for example, arginine, lysine and the like.

A particular salt form may be obtained by reacting the base form of the compound ketanserin (I) with the acid form of an ACE inhibitor as defined hereinabove.

The term addition salt as used hereinabove also comprises the solvates which the compound ketanserin, the ACE inhibitors defined hereinabove as well as the salts of ketanserin with said ACE inhibitors, are able to form. Said solvates are meant to be included within the scope of the present invention. Examples of such solvates are e.g., the hydrates, alcoholates and the like.

The compositions comprising the compound ketanserin (I) and an ACE inhibitor unexpectedly show a synergistic effect on the lowering of the blood pressure. The decrease in systolic and diastolic blood pressure (SBP ; DBP) observed in combination therapy with both ketanserin (I) and an ACE inhibitor is larger than the sum of the decreases in SBP and DBP observed in monotherapy with either ketanserin or an ACE inhibitor when applied at the same rate. As a consequence a combination therapy using compositions with lower doses of each drug than used normally in monotherapy, may be effective, and occasional side-effects associated with the larger amounts used in monotherapy may be reduced or prevented altogether. Additionally, a combination therapy using a dose of each drug as used normally in monotherapy, has the advantage that an effective lowering of the blood pressure may be obtained in the often large number of patients who do not respond to conventional monotherapy.

A further advantage of the present compositions appears to be the fact that therapy with most ACE inhibitors is accompanied with mild potassium retention (hyperkalaemia). It is known that ketanserin therapy may prove unfavorable and should be avoided in patients with a low potassium blood level. This property of ketanserin limits its utility in the treatment of hypokalaemic patients and also limits possible combination therapies of ketanserin with other drugs which may provoke hypokalaemia such as, for example, antihypertensives, in particular diuretics. The mild hyperkalaemic effect of ACE inhibitors obviously enhances the safety and utility of ketanserin therapy, particularly in patients prone to hypokalaemia.

The amount of each of the active ingredients ketanserin (I) in the free base form and an ACE inhibitor in

the neutral or zwitterionic form in the synergistic compositions according to the present invention is such that a synergistic antihypertensive effect is obtained upon administration. For example, the amount of the active ingredient ketanserin (I) in said synergistic compositions may typically range from about 5 to about 150 mg, particularly from about 10 to about 80 mg and more particularly from about 20 to about 40 mg per dosage unit form. ACE inhibitors, such as those pertaining to the patents or publication cited hereinabove and particularly the ACE inhibitors specifically mentioned hereinabove, differ both in molecular weight and in activity and therefore may be administered in fairly different quantities. As a consequence, the amount of ACE inhibitor in said synergistic compositions depends on the nature of said ACE inhibitor and in general ranges from about 1 to about 300 mg, particularly from about 5 to about 150 mg and more particularly from about 10 to about 50 mg per dosage unit form.

In said synergistic compositions the molar ratio between the active ingredient ketanserin (I) in the free base form and the ACE inhibitor in the neutral or zwitterionic form may vary depending both on the nature of the ACE inhibitor and the application aimed at. The molar ratio of the active ingredient ketanserin (I) to the ACE inhibitor will be such that a synergistic antihypertensive effect is obtained and in particular may be situated between 50:1 and 1:50, more in particular between 20:1 to 1:20 or between 10:1 and 1:10, most particularly between 5:1 and 1:5. In the synergistic compositions according to the present invention wherein an ACE inhibitor with low molecular weight and moderate activity, such as, for example, captopril is used, the molar ratio of the active ingredient ketanserin (I) to said ACE inhibitor may be situated between 5:1 and 1:30, in particular between 1:1 to 1:5 and more particularly between 1:2 and 1:3, or between 1:1 and 1:1.5. If, on the contrary a potent ACE inhibitor with relatively high molecular weight, such as, for example, enalapril, lisinopril, cilazapril or ramipril is used in the synergistic compositions according to the present invention, the molar ratio of the active ingredient ketanserin (I) to said ACE inhibitor may be situated between 50:1 and 1:5, in particular between 10:1 to 1:1 and more particularly between 5:1 and 3:1.

To prepare the pharmaceutical compositions of this invention, an effective amount of the active ingredients, in acid or base addition salt form or base form, is combined in intimate admixture with a pharmaceutically acceptable carrier, which can take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment. Acid addition salts of (I) due to their increased water solubility over the corresponding base form, are obviously more suitable in the preparation of aqueous compositions.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The present invention also concerns a method of lowering the blood pressure in warm-blooded animals suffering from hypertension or in need of blood pressure reducing medication, said method comprising administering to said warm-blooded animals an antihypertensively effective amount of the compound ketanserin (I) and an ACE inhibitor as defined hereinabove. The amount of each of the active ingredients ketanserin (I) and an ACE inhibitor in the method according to the present invention is such that a synergistic antihypertensive effect is obtained upon administration to said warm-blooded animals.

The compound ketanserin (I) or a pharmaceutically acceptable acid addition salt thereof may be administered before, during or after the administration of the ACE inhibitor, provided that the time between the administration of the compound ketanserin (I) and the administration of the ACE inhibitor is such that ketanserin (I) and the ACE inhibitor are allowed to act synergistically on the lowering of the blood pressure. When simultaneous administration of the compound ketanserin (I) and an ACE inhibitor is envisaged, a composition containing both an ACE inhibitor and the compound ketanserin (I) may be particularly convenient. Or, the compound ketanserin (I) and the ACE intubitor may be administered separately in the form of suitable compositions. The latter can be prepared as described hereinabove.

The present invention also comprises products containing the compound ketanserin (I) and an ACE inhibitor as a combined preparation for simultaneous, separate or sequential use in antihypertension therapy. Such products may comprise, for example, a kit comprising a container with a suitable composition containing ketanserin (I) or a pharmaceutically acceptable acid addition salt thereof and another container containing a composition with an ACE inhibitor. Such a product may have the advantage that a physician wishing to administer antihypertension therapy, can select on the basis of the diagnosis of the patient to be treated, the appropriate amounts of each component and the sequence and timing of the administration thereof.

Those of skill in treating warm-blooded animals suffering from hypertension or warm-blooded animals in need of blood-pressure reducing medication, could easily determine the effective amounts of both active ingredients from the results presented hereinafter. In general it is contemplated that an effective daily dose of the compound ketanserin (I) or its pharmaceutically acceptable acid addition salts would be from about 0.04 mg to about 4 mg/kg bodyweight, in particular from about 0.1 mg to about 2 mg/kg bodyweight and preferably from about 0.2 to about 1 mg/kg bodyweight, in combination with an effective daily dose of an ACE inhibitor from about 0.01 mg to about 10 mg/kg bodyweight, in particular from about 0.1 mg to about 5 mg bodyweight and preferably from about 0.2 to about 2 mg/kg bodyweight. It may be appropriate to administer the required dose as two, three, four or more sub-doses at appropriate intervals throughout the day. Said sub-doses may be formulated as unit dosage forms.

The following examples are intended to illustrate and not to limit the scope of the present invention in all its aspects.

## A. Clinical Examples.

### Example 1

The effect of a combination of the compound ketanserin (I) and an ACE inhibitor, captopril, on the lowering of blood pressure was investigated in a double-blind randomized cross-over study according to a latin square design.

Adult (≧18 years) patients with moderate, uncomplicated hypertension (105 mmHg ≦ DBP ≦ 115 mmHg at the end of a 1 month placebo wash-out period) were randomly assigned to: - placebo (1 tab q.i.d.);
- placebo (1 tab b.i.d.) + ketanserin (1 tab 40 mg b.i.d.);
- placebo (1 tab b.i.d.) + captopril (1 tab 50 mg b.i.d.);
- ketanserin (1 tab 40 mg b.i.d.) + captopril (1 tab 50 mg b.i.d.).

At the end of each treatment period (1-2 hours after the last dose), blood pressure (by a mercury sphygmomanometer) and heart rate (by the palpatory method over a 30″ period) of patients seated for 10′ were recorded by the same investigator during 3 measurements at 2′ intervals.

The following table shows the difference of systolic and diastolic blood pressure (SBP, DBP) and the heart rate (HR) (mean values ± standard error) in mmHg, respectively b.p.m., between treated and untreated patients.

| | Baseline | Placebo | Placebo + Ketanserin | Placebo + Captopril | Ketanserin + Captopril |
|---|---|---|---|---|---|
| SBP | 167.92±3.51 | +3.75±4.27 | -8.33±2.33 | -8.75±1.96 | -22.08±3.61 |
| DBP | 107.5±0.97 | -1.07±1.7 | -7.92±1.3 | -8.75±1.3 | -17.08±1.89 |
| HR | 78.17±3.31 | -2.75±3.07 | -1.92±2.2 | -3.33±2.85 | -4.83±2.65 |

Example 2

The effect of a combination of the compound ketanserin (I) and enalapril on the lowering of blood pressure was investigated in a randomized double-blind parallel group study. Elderly (≧ 50 years) patients with mild to moderate, uncomplicated, essential hypertension (95 mm Hg ≦ DBP ≦ 105 mm Hg at the end of a 3 month period on double blind monotherapy) received ketanserin (1 tab 40 mg b.i.d.) + enalapril (1 tab 10 mg b.i.d.). The results of the preliminary 3 month monotherapy phase(Ketanserin : 2 weeks, 1 tab 20 mg b.i.d., 10 weeks, 1 tab 40 mg b.i.d. or Enalapril: 12 weeks, 1 tab 10 mg b.i.d.) and the combination phase (4 weeks) are shown in the table below (16 patients). Bloodpressure (SBP, DBP) and heart rate (HR) (mean values ± standard error) are in mm Hg and b.p.m.

| | End Placebo runin | End Monotherapy Phase | | End Combination Phase | |
|---|---|---|---|---|---|
| | | Ketanserin (9 pts) | Enalapril (7 pts) | Ketanserin + Enalapril | |
| | | | | (9 pts) | (7 pts) |
| SBP | 163.12±11.52 | 148.88±13.64 | 160.0±5.77 | 144.44±9.82 | 152.85±4.87 |
| DBP | 109.06±5.23 | 102.22±5.65 | 101.42±10.07 | 89.44±7.26 | 90.71±3.45 |
| HR | 74.68±5.37 | 74.44±4.36 | 75.42±6.21 | 74.66±4.27 | 74.42±5.53 |

B. Composition Examples.

Typical pharmacological formulations in dosage unit form suitable for administration to animal and human subjects in accordance with the present invention, are those formulations described in U.S. Pat. No. 4,335,127 for the compound ketanserin (I) and those formulations wherein the active ingredient is an ACE inhibitor pertaining to one of the patents cited hereinabove, more particularly those wherein the active ingredient is one of the agent specifically mentioned hereinabove, all said patents being incorporated herein by reference for the description of said formulations. Especially convenient formulations are those formulations of the active ingredients which are commercially available.

The following formulations exemplify typical new pharmaceutical compositions in dosage unit form comprising both the compound ketanserin (I) and captopril, suitable for administration to animal and human subjects in accordance with the present invention.

Example 2 : Capsules

The following formulation provides 1000 capsules each comprising as the active ingredient 10 mg of the compound ketanserin (I) and 10 mg of captopril as defined hereinabove:

| | |
|---|---|
| ketanserin : | 10 grams |
| captopril : | 10 grams |
| sodium lauryl sulfate : | 6 grams |
| corn starch : | 56 grams |
| lactose : | 56 grams |
| colloidal silicon dioxide : | 0.8 grams |
| magnesium stearate : | 1.2 grams |

All ingredients are mixed together well. The resulting mixture is filled into suitable hardened gelatine capsules.

Example 3 : Film-coated tablets

10,000 compressed tablets, each containing as the active ingredients 20 mg of the compound ketanserin (I) and 25 mg of captopril, may be prepared from the following formulation:

| | |
|---|---|
| ketanserin : | 200 grams |
| captopril : | 250 grams |
| starch : | 400 grams |
| lactose : | 890 grams |
| polyvinylpyrollidone (Kollidon-K 90®) : | 20 grams |
| microcrystalline cellulose (Avicel®) : | 200 grams |
| sodium dodecyl sulfate : | 10 grams |
| hydrogenated vegetable oil : | 30 grams |

A mixture of the active ingredients ketanserin (I) and captopril, the lactose and the starch is mixed by vigorous stirring and is thereafter humidified with a solution of sodium dodecyl sulfate and the polyvinylpyrollidinone in about 200 ml of purified water. The wet powder is sieved, dried and sieved again. Then there is added the microcrystalline cellulose and the hydrogenated vegetable oil. The whole is mixed well and compressed into tablets. The tablets are coated with a solution methyl cellulose (Methocel) including a colour, following art-known coating procedures.

Example 4 : Injectable solution

1 liter of a parenteral solution comprising 5 mg/ml of ketanserin and 10 mg/ml of captopril may be obtained from:

| | |
|---|---|
| ketanserin : | 5 grams |
| captopril : | 10 grams |
| propylene glycol : | 0.05 grams |
| methyl 4-hydroxybenzoate : | 1.8 grams |
| propyl 4-hydroxybenzoate : | 0.2 grams |
| purified water for injection q.s. ad 1 liter. | |

The methyl and propyl 4-hydroxybenzoates are dissolved in about 0.5 l of boiling water for injection. The solution is cooled to about 50 °C and the active ingredients and the propylene glycol are added while stirring. The solution is allowed to cool to room temperature and is supplemented with water for injection q.s. ad 1 liter. The solution is sterilized by filtration (U.S.P. XVII, p 811) and filled in sterile containers.

## Claims

1. A pharmaceutical composition comprising a pharmaceutically acceptable carrier and as active ingredients the compound ketanserin or a pharmaceutically acceptable acid addition salt form thereof and an ACE inhibitor or a pharmaceutically acceptable acid or base addition salt form thereof in a quantity producing an antihypertensive synergistic effect.

2. A composition according to claim 1 wherein the ACE inhibitor is selected from benazepril, captopril, enalapril, enalaprilat, lisinopril, alacepril, delapril moveltipril, ramipril, ramiprilat, perindopril, perindoprilat, quinapril, quinaprilat, cilazapril, cilazaprilat, fosinopril, indolapril, spirapril, spiraprilat and zofenopril.

3. A composition according to claim 1 wherein the ACE inhibitor is a compound of formula

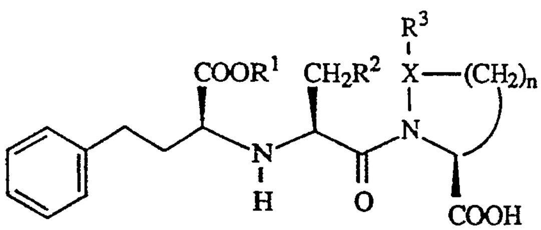

wherein $R^1$ is hydrogen or ethyl;
$R^2$ is hydrogen or aminopropyl;
$R^3$ is hydrogen; or $R^2$ and $R^3$ taken together form 1,2-ethanediyl;
X is CH or N; and
n is 2 or 3.

4. A composition according to any of claims 1 to 3 wherein the molar ratio of the compound ketanserin to the ACE inhibitor ranges from about 10:1 to about 1:10.

5. A composition according to claim 4 wherein the molar ratio ranges from about 5:1 to about 1:5.

6. A composition according to claim 1 wherein the molar ratio of ketanserin to captopril ranges from 1:1 to 1:1.5.

7. A composition according to claim 1 wherein the molar ratio of ketanserin to enalapril or to cilazapril ranges from 10:1 to 1:1.

8. A process for preparing a synergistic composition as claimed in any of claims 1 to 7, characterized in that the active ingredients are intimately mixed with a carrier.

9. A product containing the compound ketanserin or a pharmaceutically acceptable acid addition salt form thereof and an ACE inhibitor or pharmaceutically acceptable acid or base addition salt form thereof, as a combined preparation for simultaneous, separate or sequential use in antihypertension therapy.

10. A product according to claim 9 wherein the ACE inhibitor is as defined in claims 2 or 3.

European Patent Office

# EUROPEAN SEARCH REPORT

Application Number

EP 90 20 0719

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | PHARMACOLOGY, vol. 83, no. 5, May 1989, pages 273-278, S. Karger AG, Basel, CH; B. MALINOWSKA et al.: "Influence of captopril on the vasopressor effect of serotonin in pithed rats" * Pages 274-276: "Results" * | 1,2,4,5 | A 61 K 45/06<br>A 61 K 37/64 //<br>(A 61 K 45/06<br>A 61 K 31:505)<br>(A 61 K 37/64<br>A 61 K 31:505) |
| A | EP-A-0 265 685 (HOECHST AG) * Claims 1-18 * | 1-10 | |
| A | ACTA CARDIOLOGICA SINICA, vol. 4, no. 4, October/December 1988, pages 341-348, Society of Cardiology, CN; Y.-A. DING: "Update: The treatment of hypertension" * Page 344, left-hand column, line 32 - right-hand column, line 17 * | 1-10 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 20-06-1990 | BERTOCCHI C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document